# EUROPEAN PATENT SPECIFICATION

(11) **EP 2 699 217 B1**
(45) Date of publication and mention of the grant of the patent: **24.01.2018**
(21) Application number: 12710958.5
(22) Date of filing: 29.03.2012
(51) Int. Cl.: A61K 8/19, A61K 8/24, A61Q 11/00

(54) **TOOTH REMINERALIZING ORAL CARE COMPOSITIONS**
ZAHNREMINERALISIERENDE MUNDPFLEGEZUSAMMENSETZUNGEN
COMPOSITIONS DE SOINS BUCCO-DENTAIRES PERMETTANT LA REMINÉRALISATION DES DENTS

(30) Priority: 18.04.2011 WO PCT/CN2011/000667
(43) Date of publication of application: 26.02.2014
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); DENG, Yan, Shanghai 200335 (CN); DING, Guan-Jun, Shanghai 200050 (CN); XU, Yong, Shanghai 201203 (CN)
(74) Representative: Whaley, Christopher
(86) International application number: PCT/EP2012/055604
(87) International publication number: WO 2012/143220

(56) References cited:
- WO-A1-00/10520
- WO-A1-99/47108
- WO-A1-2008/068149
- US-A- 4 083 955
- US-A1- 2003 072 721
- US-A1- 2008 050 408
- US-A1- 2008 292 565

## Description

### FIELD OF THE INVENTION

The present invention is directed to a tooth-remineralizing oral care composition as well as a method for remineralizing teeth. More particularly the present invention relates to such compositions and methods which employ calcium salt having a weight average particle size of 0.01 to 3 microns.

### BACKGROUND OF THE INVENTION

Products we enjoy as consumers, unfortunately, often have a negative impact on our teeth. Acidic drinks and sweets, for example, can result in tooth erosion by attacking enamel that coats and protects the teeth. Moreover, tobacco based products as well as beverages like coffee and tea can stain teeth and thereby result in a smile that is often not attractive.

In addition to what we consume, the natural equilibrium between tooth hydroxyapatite being dissolved from the enamel of teeth and hydroxyapatite being formed on or in teeth from substances occurring naturally in the saliva shifts continuously. Such a shift can yield unattractive teeth with cariogenic conditions. Products that address tooth decay and/or whitening have, nevertheless, been developed. Conventional products often comprise peroxides, coarse abrasives or both. These types of products are often not desired since they can cause damage to teeth and gums if improperly used. Moreover, such products can be expensive and are not attractive to lower income consumers in need of tooth remineralization.

We have recognized a need to develop an oral care product that is suitable to whiten and remineralize teeth while at the same time being gentle for use and affordable for a broad range of consumers. This invention, therefore, is directed to a tooth-whitening and tooth-remineralizing oral care composition as well as a method for remineralizing teeth. The oral care composition of this invention having an average particle size of 0.01 to 3 microns microns unexpectedly results in an excellent component for *in-situ* hydroxyapatite formation. The composition may additionally or alternatively result in whiter and remineralized teeth after use wherein the composition is gentle on teeth of a consumer.

Efforts have been disclosed for making oral care compositions. In WO 00/10520 and US 2003/0072721 A1, compositions with fine calcium carbonate and fine ground natural chalk, respectively, are described.

Other efforts have been disclosed for making oral care compositions. In PCT Applications published as WO 2008/068149 A1 and WO 2008/068248 A1; and United States Patent Nos. 4,083,955, 5,605,675 and 6,214,321, processes and compositions for remineralizing dental enamel are described.

Still other efforts have been disclosed for making oral care compositions. In U.S. Patent Application published as US 2007/0264291 A1, compositions and methods for preventing or reducing plaque and/or gingivitis using a bioactive glass containing dentifrice are described.

Even other efforts have been disclosed for making oral care compositions. In U.S. Application published as US 2008/292565 A1, tooth fluoridating and remineralizing compositions are described.

None of the publications summarized above describes an oral care composition wherein the composition comprises calcium salt having a weight average particle size of 0.01 to 3 microns wherein, the same is an excellent component for *in situ* hydroxyapatite formation.

### SUMMARY OF THE INVENTION

In a first aspect, the present invention is directed to an oral care composition, the oral care composition comprising:
a) from 5 to 45% by weight calcium salt having a weight average particle size of 0.01 to 3 microns and
b) from 0.01 to 30% by weight of a phosphate source,
   the calcium salt and phosphate source, together, being precursors for *in situ* hydroxyapatite formation.

In a second aspect, the present invention is directed to whitening and remineralizing teeth with the oral care composition of the first aspect of this invention.

All other aspects of the present invention will more readily become apparent upon considering the detailed description and examples which follow.

Regeneration-source calcium salt, as used herein means the calcium salt having a weight average particle size of 0.01 to 3 and preferably, from 0.02 to 1.5 microns whereby particle size is meant to mean the largest measurable size of the particle and including the diameter thereof. In an especially preferred embodiment, from 1 to 100% by weight of the regeneration-source calcium salt used in this invention has a weight average particle size from 0.1 to 2 microns. Such regeneration-source calcium salt is provided to yield tooth remineralization *in situ* and along with phosphate source.

Oral care composition means a composition suitable for use in veterinary and/or human oral cavity applications but especially for use in a human oral cavity application. Such a composition may be single phase or the result of a mixture of a calcium and phosphate phase. Soluble and insoluble, as used herein, refers to the solubility of a source (e.g., like calcium salts) in water. Soluble means a source that dissolves in water to give a solution with a concentration of at least 0.1 moles per liter at room temperature (25 °C). Insoluble means a source that dissolves in water to give a solution with a concentration of less than 0.001 moles per liter at room temperature. Slightly soluble, therefore, is defined to mean a source that dissolves in water to give a solution with a concentration of greater than 0.001 moles per liter at room temperature and less than 0.1 moles per liter at room temperature. Remineralization, as used herein, means *in situ* generation of hydroxyapatite on teeth (including layers on teeth from 10 nm to 6 microns, and preferably from 75 nm to 5 microns, and most preferably, from 150 nm to 4 microns thick including all ranges subsumed therein) to reduce the likelihood of tooth sensitivity, tooth decay, regenerate enamel and/or improve the appearance of teeth by whitening through the generation of such new hydroxyapatite. New hydroxyapatite, including layers thereof, typically covers at least 30 percent, and preferably, at least 45 percent and most preferably from 48 to 100 percent of the surface of teeth subjected to the oral care composition of this invention and including all ranges subsumed therein. Whitening can also include the physical whitening of teeth through the adhesion of calcium-based salts and other opacifiers temporarily to the surface of teeth. Oral care composition is meant to include, for example, a powder, gel, liquid (like mouthwash), spray, foam, balm, composition carried or a mouthstrip or a buccal adhesive patch, chewable tablet (or pastille), lozenge, cream, beverage or a strip of gum. Preferably, however, the composition is a paste-like toothpaste or a gel for teeth.

All ranges are meant to include all ranges subsumed therein. Comprising, as used herein, is meant to include consisting essentially of and consisting of. For the avoidance of doubt, the hydroxyapatite precursors in the composition may consist essentially of or consist of calcium salt having a weight average particle size of 0.01 to 3 microns and phosphate source.

### DETAILED DESCRIPTION

The only limitations with respect to the calcium salt having a weight average particle size of 0.01 to 3 microns that may be used in this invention is that the same is suitable for use in oral care compositions.

Illustrative yet non-limiting examples of the types of calcium salt that may be used in this invention include those which are water insoluble like calcium carbonate, calcium silicate, calcium phosphate, calcium gluconate, calcium oxide, calcium lactate, calcium hydroxide, calcium sulfate, calcium carboxymethyl cellulose, calcium alginate, calcium salts of citric acid, mixtures thereof or the like. In a preferred embodiment, the calcium salt comprises at least 80 percent by weight calcium carbonate based on total weight of the salt. Most preferably, the calcium salt is from 90 to 100 percent by weight calcium carbonate.

When a calcium silicate is employed, the same may comprise calcium oxide-silica (CaO-SiO₂) as described in commonly-owned PCT applications published as WO 2008/015117 and WO 2008/068248. The oral care composition of the present invention comprises from 5 to 45% by weight calcium salt having a weight average particle size of 0.01 to 3 microns and preferably, from 15 to 35% by weight of the regeneration-source calcium salt, based on total weight of the composition and including all ranges subsumed therein. In a preferred embodiment, from 10 to 100%, and especially, from 25 to 100%, and most especially, from 70 to 100% by weight of the regeneration-source calcium salt used in this invention has a particle size from 0.1 to 1.5 microns. Such calcium salts suitable for use in this invention are commercially available and often sold commercially from suppliers like Cole-Parmer, Great Lakes Calcium and Fujian Sannong Calcium Carbonate Co., Ltd.

The phosphate source suitable for use in this invention is one which may be used in an oral care composition and able to provide phosphate to form *in situ* hydroxyapatite with the calcium supplied from the regeneration-source calcium salt used. Illustrative examples of the types of phosphate source suitable for use in this invention include sodium dihydrogen phosphate, disodium hydrogen phosphate, sodium pyrophosphate, tetrasodium pyrophosphate, sodium tripolyphosphate, sodium hexametaphosphate, potassium dihydrogen phosphate, dipotassium hydrogen phosphate, tripotassium phosphate, mixtures thereof or the like. The phosphate source is preferably one which is water soluble.

Typically, the phosphate source makes up from 0.5 to 25%, and preferably, from 2 to 20%, and most preferably, from 4 to 16% by weight of the oral care composition, based on total weight of the oral care composition and including all ranges subsumed therein.

In a preferred embodiment, the phosphate source used is one which results in an oral care composition having a pH from 5.5 to 10, and preferably, from 6 to 8, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably, 1:3 to 3:1, and most preferably, from 1:2 to 2:1, including all ratios subsumed therein.

Oral care compositions for whitening and remineralizing teeth and comprising the regeneration-source calcium salt described herein may comprise optional ingredients which are common in the art. These ingredients include:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and strontium polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc.;
▪ biomolecules, e.g. bacteriocins, antibodies, enzymes, etc.
▪ flavors, e.g., peppermint and spearmint oils;
▪ proteinaceous materials such as collagen;
▪ preservatives;
▪ opacifying agents (especially titanium dioxide);
▪ coloring agents like FD&C blue, yellow and/or red dyes/colorants;
▪ pH-adjusting agents;
▪ sweetening agents;
▪ surfactants, such as anionic, nonionic, cationic and zwitterionic or amphoteric surfactants (e.g., sodium lauryl sulfate, sodium dodecylbenzene sulfonate);
▪ particulate abrasive materials such as abrasive silicas, aluminas, abrasive calcium salts like calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643;
▪ buffers and salts to buffer the pH and ionic strength of the oral care compositions; and
▪ other optional ingredients that may be included are, e.g., bleaching agents such as peroxy compounds, e.g., potassium peroxydiphosphate, effervescing systems such as sodium bicarbonate/citric acid systems, color change systems, and the like.

Such ingredients typically and collectively make-up less than 20% by weight of the oral care composition comprising the regeneration-source calcium salt described herein, and preferably, from 0.0 to 15% by weight, and most preferably, from 0.01 to 12% by weight of the oral care composition, including all ranges subsumed therein. Calcium salt having a weight average particle size of greater than 5 microns in at least one dimension may be employed in this invention along with the regeneration-source calcium salt described herein.

In another especially preferred embodiment, from 0.05 to 7%, and most preferably, from 1.5 to 5.5 % by weight TiO₂ is present in the oral care composition based on total weight of the composition and including all ranges subsumed therein.

Thickener may also be used in this invention and is limited only to the extent that the same may be added to a composition suitable for use in the mouth along with the regeneration-source calcium salt of this invention. Illustrative examples of the types of thickeners that may be used in this invention include, sodium carboxymethyl cellulose, hydroxyl ethyl cellulose, methyl cellulose, ethyl cellulose, gum tragacanth, gum Arabic, gum karaya, sodium alginate, carrageenan, guar, xanthan gum, Irish moss, starch, modified starch, silica based thickeners including silica aerogels, magnesium aluminum silicate (i.e., Veegum) Carbomers (cross-linked acrylates) and mixtures thereof.

Typically, sodium carboxymethyl cellulose and/or a Carbomer is/are preferred. When a Carbomer is employed, those having a molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, and preferably, from 0.1 to 8%, and most preferably, from 1.5 to 6% by weight of the oral care composition, based on total weight of the oral care composition and including all ranges subsumed therein.

Suitable carriers that may be employed in this invention are, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carriers should, in any case, be substantially free of water, and preferably, anhydrous if a single phase product suitable for tooth remineralization is desired and the same comprises both a phosphate as an additive and the regeneration-source calcium salt described herein as hydroxyapatite precursors. The carrier can, for example, be used in solid form, but glycerin is often the preferred carrier or humectant in single phase products.

The carrier is used to take the balance of the single phase compositions up to 100%, and the same may be present in the range from 10 to 90% by weight of the single phase oral care composition. Preferably, carrier makes up from 25 to 80%, and most preferably, from 45 to 70% by weight of the single phase oral care composition, based on total weight of the single phase oral care composition and including all ranges subsumed therein.

In the event an aqueous oral care composition suitable for tooth whitening and remineralization is desired, a dual phase oral care composition is recommended to prevent interaction (prior to use) between the phosphate source and regeneration-source calcium salt.

When a dual phase oral care composition is desired, water may act as a carrier (along with thickeners and/or additional carriers herein described) and make up the balance of each composition in the dual phase product wherein a first composition will comprise regeneration-source calcium salt and a second composition will comprise the added phosphate source. Within the dual phase product, the first and second compositions (or phases) should not come into contact with each other until dispensed for use by the consumer in his or her mouth. When a dual phase oral care composition is used, the weight percents described herein are meant to characterize the oral care composition after the first and second compositions have been combined. The delivery of each composition (in the event a dual composition product is desired) may be sequential or simultaneous, but preferably, simultaneous. In a preferred embodiment, each composition within the dual phase oral care composition comprises less than 35% by weight water, and most preferably, from 15 to 25% by weight water, based on total weight of each composition and including all ranges subsumed therein. Each composition of the dual phase product is typically used at a weight ratio from 40:60 to 60:40, but preferably, at a 55:45 to 45:55 weight ratio, and most preferably, at a 50:50 weight ratio.

One or preferably both of the compositions within the dual phase oral care composition may be applied to the teeth, with treatment of the teeth involving mixing of the compositions. Whether single or dual phase, the compositions are preferably left on the teeth following application. Following such application, the oral care compositions of this invention should typically be left on the teeth for 5 seconds to 10 hours and more typically from 35 seconds to 30 minutes. The application may be carried out daily. If a dual composition is employed, the same may be applied from independent compartments at a dual compartment tube or from independent phases of a product contained within a single container which is typically a tube.

In certain embodiments, in particular those involving a gel composition, the means of delivery may optionally involve a tape, in particular an adhesive tape strip, onto which the compositions of this invention are applied prior to the strip being placed in contact with the teeth. When using such a means of delivery, the compositions can be held in close contact with the surface of the teeth, facilitating a high concentration of composite particle active and phosphate close to the surface of the teeth.

When a gel is desired, the same typically comprises a polymeric matrix, and is more typically a hydrogel. Excluding any water present, the polymeric matrix is typically present at from 1 to 25% by weight of the composition of which it is a part.

Monomers used to prepare hydrogels may be selected from, for example, vinyl alcohol and acrylate, in particular sodium acrylate. Other monomers comprising an abundance of hydrophilic groups may also be used.

Often preferred hydrogels comprise a polysaccharide, polyacrylamide, polyacrylic acid, or a mixture thereof.

Suitable polysaccharides may be storage polysaccharides, such as starch or glycogen, or structural polysaccharides, such as cellulose or chitin.

Illustrative polysaccharides which may be used include those having saccharide units selected from one or more of the following: isomaltose, glucose, fructose, galactose, xylose, mannose, sorbose, arabinose, rhamnose, fucose, maltose, sucrose, lactose, maltulose, ribose, lyxose, allose, altrose, gulose, idose, talose, trehalose, nigerose, kojibiose, and lactulose.

Often preferred hydrogels comprise at least one polysaccharide selected from the group consisting of: tamarind gum, guar gum, locust bean gum, Tara, Fenugreek, Aloe, Chia, Flaxseed, Psyllium seed, quince seed, xanthan, gellan, welan, rhamsan, dextran, curdlan, pullulan, scleroglucan, schizophyllan, chitin, hydroxyalkyl cellulose, arabinan, de-branched arabinan, arabinoxylan, glactan, pectic galactan, galactomannan, glucomannan, lichenan, mannan, pachyman, rhamnogalacturonan, acacia gum, agar, alginates, carrageenan, chitosan, clavan, hyaluronic acid, heparin, inulin, cellodextrins, cellulose, and cellulose derivatives.

Especially preferred hydrogels can comprise polysaccharides like those selected from the group consisting of: sodium alginate, hydroxypropyl alginate, gum carrageenan, gum arabic, guar gum, karaya gum, chitosan and pectin.

Compositions comprising the regeneration-source calcium salt described herein (whether single or dual phase) are prepared by conventional methods of making oral care compositions. Such methods include mixing the ingredients under moderate shear and atmospheric pressure. The compositions are desired for use in the mouth, and preferably, are of the form that may be brushed onto teeth with a toothbrush. Unexpectedly, the oral care compositions of this invention result in excellent remineralization of teeth (i.e., new hydroxyapatite formation) and teeth whitening (which may be immediate and provided for by the regeneration-source calcium salt and opacifiers used) as a result of oral care composition coming into contact with enamel and/or dentin of teeth. Moreover, subsequent to using the compositions of this invention, teeth are preferably less sensitive, and/or shinier, the same also being a direct result of hydroxyapatic formation *in situ.*

In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. Solid dosage form types include pastilles, lozenges, chewing gums, tablets, mouthstrips, balms, and the like. These may be contained in conventional packaging desirable for consumer use.

The composition may be used for providing at least one benefit to the teeth of an individual, said benefit selected from remineralization, decreased sensitivity, whitening and combinations thereof. The composition may also be used in the manufacture of a medicament for providing at least one of said benefits. The composition may also be for use in providing at least one of said benefits. Said uses preferably comprise administering the composition to the surface of the teeth of the individual.

The following examples are provided to facilitate an understanding of the present invention. The examples are not intended to limit the scope of the claims.

### Example 1

A dual phase aqueous-based oral care composition capable of remineralizing teeth and consistent with this invention was made by mixing the ingredients below under conditions of moderate shear, atmospheric pressure and ambient temperature. The compositions made were suitable for use with a toothbrush, and when combined about equally were not gritty and resulted in an excellent ribbon when applied to a toothbrush. The combined compositions, which resulted in the oral care composition of this invention, had consumer acceptable taste characteristics.

**Calcium Comprising Phase: SAMPLE**

| Ingredients | 1 (% by weight) | 2 (% by weight) | 3 (% by weight) |
|---|---|---|---|
| Sorbitol (70%) | 20.0 | 20.0 | 20.0 |
| Water | Balance | balance | balance |
| Preservative | 0.5 | 0.5 | 0.5 |
| Sweetener (artificial) | 0.2 | 0.2 | 0.2 |
| Regeneration-source calcium carbonate* | 30.0 | 30.0 | 30.0 |
| Thickening silica (fumed silica) | 3.5 | 3.0 | 3.5 |
| Abrasive silica | 4 | 4 | 4 |
| TiO₂ | 0 | 4 | 2 |
| Flavor | 1.0 | 1.0 | 1.0 |
| Sodium carboxymethyl cellulose | 0.6 | 0.5 | 0.5 |
| Sodium lauryl sulphate (30%) | 6.6 | 6.6 | 6.6 |

| | | | |
|---|---|---|---|
| * particle diameter between 0.1-2 microns | | | |

**Phosphate Comprising Phase:**

| Ingredients | Percent by Weight |
|---|---|
| Sorbitol (70%) | 55.0 |
| Trisodium phosphate | 7.7 |
| Water | balance |
| Polyethylene glycol (1500) | 2.0 |
| Sweetener (artificial) | 0.27 |
| Abrasive silica | 12.0 |
| Monosodium phosphate | 6.4 |
| Thickening silica (fumed silica) | 3.5 |
| Sodium lauryl sulphate 30% | 6.6 |
| Flavor | 1.0 |
| Sodium carboxymethyl cellulose | 0.5 |

### Example 2

The calcium comprising phase of this example was made in a manner similar to the one described in Example 1 except that 40% by weight calcium carbonate (diameter 30 microns) was used in lieu of regeneration-source calcium carbonate. The phosphate comprising phase in this example was made in essentially the same manner described for the phosphate phase generation in Example 1.

**Calcium Comprising Phase**

| Ingredients | Percent by Weight |
|---|---|
| Sorbitol (70%) | 20/0 |
| Water | Balance |
| Preservative | 0.5 |
| Sweetener (artificial) | 0.2 |
| Calcium carbonate* | 40.0 |
| Thickening silica (fumed silica) | 3.5 |
| Flavor | 1.0 |
| Sodium carboxymethyl cellulose | 0.6 |
| Sodium lauryl sulphate (30%) | 6.6 |

| | |
|---|---|
| *weight average particle size 30 microns | |

### Example 3

To investigate the remineralization effect of the dual phase oral care compositions of Examples 1 and 2, the following *in vitro* test was performed.

Extracted human cadaver teeth were cleaned by washing the same with a 75% ethanol wash and scraping the teeth to remove calculus and/or stains until no visible surface dirt or stains remained on the surface of the teeth. Eight tooth blocks were used in each group tested. All groups were well hydrated in water at least one (1) day before testing. A calcium salt phase slurry and a phosphate salt phase slurry were prepared separately by mixing toothpaste and water at a ratio of 1:2. Tooth samples were manually brushed for two (2) minutes in a 10 mL mixture of slurry (5 mL calcium slurry and 5 mL phosphate slurry, brushed immediately once mixed), subsequently washed with distilled water and stored in simulated oral fluid at 37°C for two (2) hours. After two hours of storage, the groups of teeth were taken out, and brushed again in a similar fashion. This procedure was repeated 28 times (twice daily and over two (2) weeks). Simulated oral fluid was made by combining the following:

**Simulated Oral Fluid**

| Ingredient | Amount |
|---|---|
| NaCl | 16.1g |
| NaHCO₃ | 0.7g |
| KCl | 0.4g |
| K₂HPO₃*H₂O | 3.3g |
| MgCl₂*6H₂O | 0.6g |
| 1M HCl | 40 mL |
| CaCl₂ | 0.2g |
| Na₂SO₄ | 0.14g |
| Buffer | Adjust pH to 7.0 |
| Water | Balance to two liters |

The treatments to the teeth of Group II were the same as those for Group I except that the compositions of Example 2 were used in lieu of the compositions prepared in Example 1.

Calcium carbonate particle deposition on the surface of the subject teeth was evaluated by Scanning electron Microscopy (SEM) observation after a single in vitro brushing. After one (1) *in vitro* brushing treatment, the samples were rinsed with water and air dried before characterization, followed by assessment via SEM (S-4800 apparatus, Hitachi, Japan). Results unexpectedly revealed that significant calcium deposited on the surface of the teeth when a composition with regeneration-source calcium salt was used. The calcium deposition observed was calcium carbonate. For the composition with thirty (30) micron calcium salt, only a trace amount of calcium comprising particles were observed on the surface of the teeth assessed.

The remineralization effect of dual phase oral care compositions made consistent with this invention was assessed by observing new hydroxyapatite layer formation on the surface of teeth after two (2) weeks of *in vitro* brushing treatment. After two (2) weeks of brushing, the treated groups of teeth were embedded in epoxy resin, and then cut into thin slices (i.e., cross-sections) with a diamond saw. The slices were subsequently polished with an alumina slurry. In order to observe the boundary of tooth enamel and new *in situ* formed hydroxyapatite, the slices were incubated in 0.1 % citric acid solution for three (3) minutes to expose the microstructure of the enamel in the teeth. After washing with water and drying at 50°C for 24 hours, the cross-sections were observed via SEM to assess how much new enamel or hydroxyapatite layer had accumulated during the test.

Results unexpectedly revealed that, a new hydroxyapatite layer formed on the enamel surface of the teeth. The thickness of the new hydroxyapatite layer was surprisingly about 1-3 microns after the two (2) week treatment with the oral care composition of this invention. Energy dispersive x-ray (EDS) analysis showed that the new layer was essentially composed of Ca and P with Ca/P ratio of about 1.7.

Newly formed hydroxyapatite layers increase light reflection as new hydroxyapatite layer thickens. The results of the full spectrum of light reflection on the samples assessed both before and after new layer formation unexpectedly revealed that light reflection was thoroughly enhanced when new hydroxyapatite layer reached one (1) micron. Such an unexpected increase in light reflection resulted in teeth that were significantly whiter subsequent to treatment with compositions made consistent with this invention.

The results surprisingly demonstrated that the regeneration-source calcium salt comprising compositions of the present invention resulted in the whitening and remineralization of teeth, a direct result of hydroxyapatite formation.

The composition comprising the phases of Example 2 did not yield perceivable hydroxyapatite layer formation and whitening.

### Example 4

A monophase oral care composition consistent with this invention was prepared by mixing (in weight percent) the following ingredients under moderate shear until a homogeneous composition was obtained.

| Ingredient | Sample 1 (% by weight) | Sample 2 (% by weight) |
|---|---|---|
| Glycerin | Balance | Balance |
| TiO₂ | 2 | 0 |
| Calcium carbonate* | 15 | 15 |
| Silica abrasive | 8 | 8 |
| Trisodium phosphate | 3.8 | 3.8 |
| Monosodium dihydrogen phosphate | 3.2 | 3.2 |
| Sodium monofluorophosphate | 10.8 | 10.8 |
| Sodium lauryl sulfate (70%) | 2.2 | 2.2 |
| Flavor | 1 | 1 |
| Thickener (acrylate-based) | 0.3 | 0.3 |
| Silica thickener | 3 | 2 |

| | | |
|---|---|---|
| * particle diameter between 0.1-2 microns | | |

### Example 5

The monophase oral care compositions of this Example were made in a manner similar to the one described in Example 4 except that 15% by weight calcium carbonate (diameter 30 microns) was used in lieu of regeneration-source calcium carbonate consistent with this invention.

### Example 6

The investigation in Example 3 was repeated except that the monophase compositions of Examples 4 and 5 were used in lieu of the dual phases described in Examples 1 and 2 and treatment was for four (4) weeks of *in vitro* brushing treatment.

Results unexpectedly revealed that a new hydroxyapatite layer formed on the surface of the teeth assessed. The thickness of the new hydroxyapatite layer was surprisingly about 1 micron after 4 weeks of treatment with the oral care composition of this invention. EDS composition analysis showed that the new layer was predominately composed of Ca and P with Ca/P ratio of about 1.9.

The composition comprising the monophase oral care compositions of Example 5 did not yield any perceivable hydroxyapatite layer formation and whitening.

The results obtained unexpectedly reveal that excellent tooth remineralization and whitening can be achieved when oral care compositions are formulated with regeneration-source calcium salt consistent with this invention and in lieu of calcium salt having a weight average particle size greater than five (5) microns.

### Example 7

Dual phase oral care compositions similar to the one described in Example 1 were made except that 14 percent by weight sodium phosphate was used as the sole phosphate source in the phosphate phase and regeneration-source calcium salt (particle diameter between 0.1 to 2 microns) was used in the calcium phase at the levels indicated below. The two phases were used at about a 50:50 weight ratio.

| Sample | % by weight calcium carbonate |
|---|---|
| 1 | 0 |
| 2 | 20 |
| 3 | 30 |

Sixty (60) panelists participated in a consumer study. Three (3) groups of twenty (20) were set up where the first group used the composition of Sample 1, the second group used the composition of Sample 2 and the third group used the composition of Sample 3. Teeth of the panelists were evaluated before the study commenced. Initial luminosity (L) was assessed with a conventional digital imaging apparatus. Final luminosity (L) was assessed after all panelists brushed their teeth twice per day for four (4) weeks wherein the panelists were instructed to brush their teeth as they normally do.

The results revealed no improvement to the teeth of panelist using sample 1. Use of sample 2 during the period resulted in a slight improvement but use of sample 3 during the period resulted in a significant improvement to the luminosity (L) of the treated teeth.

Such results unexpectedly reveal that use of oral care compositions consistent with this invention will result in excellent whitening benefits to teeth of consumers.

## Claims

1. An oral care composition comprising:
a) from 5 to 45% by weight calcium salt having a weight average particle size of 0.01 to 3 microns; and
b) from 0.01 to 30% by weight soluble phosphate source,
the calcium salt and soluble phosphate source, together, being precursors for *in-situ* hydroxyapatite formation on teeth.

2. The oral care composition according to claim 1 wherein the calcium salt is calcium carbonate, calcium silicate, calcium sulfate, calcium phosphate, calcium gluconate, calcium oxide, calcium lactate, calcium hydroxide, calcium carboxymethyl cellulose, calcium alginate, calcium salts of citric acid, or a mixture thereof.

3. The oral care composition according to claim 1 or claim 2 wherein the calcium salt comprises at least 80% by weight calcium carbonate based on total weight of calcium salt.

4. The oral care composition according to any one of claims 1 to 3 wherein the oral care composition comprises from 15 to 35% by weight calcium salt.

5. The oral care composition according to any one of the preceding claims wherein the precursors in the oral care composition for *in-situ* hydroxyapatite formation or teeth consist essentially of calcium salt and phosphate source.

6. The oral care composition according to any of the preceding claims wherein the calcium salt salt has a weight average particle size of 0.02 to 1.5 microns.

7. The oral care composition according to claim 6 wherein from 10 to 100% by weight of the total weight of calcium salt used has a weight average particle size from 0.1 to 1.5 microns.

8. The oral care composition according to claim 7 wherein from 25 to 100% by weight of the total weight of calcium salt used has a weight average particle size from 0.1 to 1.5 microns.

9. The oral care composition according to claim 8 wherein from 70 to 100% by weight of the total weight of calcium salt used has a weight average particle size from 0.1 to 1,5 microns.

10. The oral care composition according to any one of the preceding claims wherein the phosphate source comprises sodium dihydrogen phosphate, disodium hydrogen phosphate, sodium pyrophosphate, tetrasodium pyrophosphate, sodium tripolyphosphate, sodium hexametaphosphate, potassium dihydrogen phosphate, dipotassium hydrogen phosphate, tripotassium phosphate or a mixture thereof.

11. The oral care composition according to any one of the preceding claims wherein the phosphate source makes up from 0.5 to 25% by weight of the oral care composition.

12. The oral care composition according to any one of the preceding claims wherein the composition further comprises from 0.05 to 7% by weight TiO₂.

13. A composition according to any one of claims 1 to 12 for use in the treatment of remineralization and/or desensitization of teeth.

14. Use of a composition according to any one of the preceding claims for the whitening of teeth.

## Patentansprüche

1. Mundpflegezusammensetzung, umfassend:
a) von 5 bis 45 Gewichts-% Calciumsalz einer gewichtsgemittelten Partikelgröße von 0,01 bis 3 Mikron und
b) von 0,01 bis 30 Gewichts-% lösliche Phosphat-Quelle,
wobei das Calciumsalz und die lösliche Phosphat-Quelle, zusammen, Vorstufen für eine *in-situ*-Hydroxyapatit-Bildung auf Zähnen sind.

2. Mundpflegezusammensetzung nach Anspruch 1, wobei das Calciumsalz Calciumcarbonat, Calciumsilikat, Calciumsulfat, Calciumphosphat, Calciumgluconat, Calciumoxid, Calciumlaktat, Calciumhydroxid, Calciumcarboxymethylcellulose, Calciumalginat, Calciumsalze von Zitronensäure oder eine Mischung davon darstellt.

3. Mundpflegezusammensetzung nach Anspruch 1 oder Anspruch 2, wobei das Calciumsalz mindestens 80 Gewichts-% Calciumcarbonat, bezogen auf das Gesamtgewicht des Calciumsalzes, umfasst.

4. Mundpflegezusammensetzung nach irgendeinem der Ansprüche 1 bis 3, wobei die Mundpflegezusammensetzung von 15 bis 35 Gewichts-% Calciumsalz umfasst.

5. Mundpflegezusammensetzung nach irgendeinem der vorhergehenden Ansprüche, wobei die Vorstufen in der Mundpflegezusammensetzung für die *in-*situ-Hydroxyapatit-Bildung auf Zähnen im Wesentlichen aus Calciumsalz und Phosphat-Quelle bestehen.

6. Mundpflegezusammensetzung nach irgendeinem der vorhergehenden Ansprüche, wobei das Calciumsalz eine gewichtsgemittelte Partikelgröße von 0,02 bis 1,5 Mikron aufweist.

7. Mundpflegezusammensetzung nach Anspruch 6, wobei von 10 bis 100 Gewichts-% des Gesamtgewichts des verwendeten Calciumsalzes eine gewichtsgemittelte Partikelgröße von 0,1 bis 1,5 Mikron aufweisen.

8. Mundpflegezusammensetzung nach Anspruch 7, wobei von 25 bis 100 Gewichts-% des Gesamtgewichts des verwendeten Calciumsalzes eine gewichtsgemittelte Partikelgröße von 0,1 bis 1,5 Mikron aufweisen.

9. Mundpflegezusammensetzung nach Anspruch 8, wobei von 70 bis 100 Gewichts-% des Gesamtgewichts des verwendeten Calciumsalzes eine gewichtsgemittelte Partikelgröße von 0,1 bis 1,5 Mikron aufweisen.

10. Mundpflegezusammensetzung nach irgendeinem der vorhergehenden Ansprüche, wobei die Phosphat-Quelle Natriumdihydrogenphosphat, Dinatriumhydrogenphosphat, Natriumpyrophosphat, Tetranatriumpyrophosphat, Natriumtripolyphosphat, Natriumhexametaphosphat, Kaliumdihydrogenphosphat, Dikaliumhydrogenphosphat, Trikaliumphosphat oder eine Mischung davon umfasst.

11. Mundpflegezusammensetzung nach irgendeinem der vorhergehenden Ansprüche, wobei die Phosphat-Quelle 0,5 bis 25 Gewichts-% der Mundpflegezusammensetzung ausmacht.

12. Mundpflegezusammensetzung nach irgendeinem der vorhergehenden Ansprüche, wobei die Zusammensetzung ferner von 0,05 bis 7 Gewichts-% TiO₂ umfasst.

13. Zusammensetzung nach irgendeinem der Ansprüche 1 bis 12 zur Verwendung in der Behandlung der Remineralisation und/oder Desensibilisierung von Zähnen.

14. Verwendung einer Zusammensetzung nach irgendeinem der vorhergehenden Ansprüche zum Aufhellen von Zähnen.

## Revendications

1. Composition pour le soin oral comprenant :
a) de 5 à 45 % en masse de sel de calcium ayant une taille moyenne de particule en masse de 0,01 à 3 microns ; et
b) de 0,01 à 30 % en masse de source de phosphate soluble,
le sel de calcium et la source de phosphate soluble, ensemble, étant des précurseurs pour une formation *in-situ* d'hydroxyapatite sur des dents.

2. Composition pour le soin oral selon la revendication 1, dans laquelle le sel de calcium est le carbonate de calcium, le silicate de calcium, le sulfate de calcium, le phosphate de calcium, le gluconate de calcium, l'oxyde de calcium, le lactate de calcium, l'hydroxyde de calcium, la calcium carboxyméthylcellulose, l'alginate de calcium, des sels de calcium d'acide citrique, ou un mélange de ceux-ci.

3. Composition pour le soin oral selon la revendication 1 ou la revendication 2, dans laquelle le sel de calcium comprend au moins 80 % en masse de carbonate de calcium rapporté à la masse totale de sel de calcium.

4. Composition pour le soin oral selon l'une quelconque des revendications 1 à 3, dans laquelle la composition pour le soin oral comprend de 15 à 35 % en masse de sel de calcium.

5. Composition pour le soin oral selon l'une quelconque des revendications précédentes, dans laquelle les précurseurs dans la composition pour le soin oral pour une formation *in-situ* d'hydroxyapatite sur des dents sont essentiellement constitués de sel de calcium et de source de phosphate.

6. Composition pour le soin oral selon l'une quelconque des revendications précédentes, dans laquelle le sel de calcium présente une taille moyenne de particule en masse de 0,02 à 1,5 microns.

7. Composition pour le soin oral selon la revendication 6, dans laquelle de 10 à 100 % en masse de la masse totale de sel de calcium utilisé présente une taille moyenne de particule en masse de 0,1 à 1,5 microns.

8. Composition pour le soin oral selon la revendication 7, dans laquelle de 25 à 100 % en masse de la masse totale de sel de calcium utilisé présente une taille moyenne de particule en masse de 0,1 à 1,5 microns.

9. Composition pour le soin oral selon la revendication 8, dans laquelle de 70 à 100 % en masse de la masse totale de sel de calcium utilisé présente une taille moyenne de particule en masse de 0,1 à 1,5 microns.

10. Composition pour le soin oral selon l'une quelconque des revendications précédentes, dans laquelle la source de phosphate comprend le dihydrogénophosphate de sodium, l'hydrogénophosphate de disodium, le pyrophosphate de sodium, le pyrophosphate de tétrasodium, le tripolyphosphate de sodium, l'hexamétaphosphate de sodium, le dihydrogénophosphate de potassium, l'hydrogénophosphate de dipotassium, le phosphate de tripotassium ou un mélange de ceux-ci.

11. Composition pour le soin oral selon l'une quelconque des revendications précédentes, dans laquelle la source de phosphate constitue de 0,5 à 25 % en masse de la composition pour le soin oral.

12. Composition pour le soin oral selon l'une quelconque des revendications précédentes, dans laquelle la composition comprend de plus de 0,05 à 7 % en masse de TiO₂.

13. Composition selon l'une quelconque des revendications 1 à 12 pour une utilisation dans le traitement de reminéralisation et/ou de désensibilisation de dents.

14. Utilisation d'une composition selon l'une quelconque des revendications précédentes pour le blanchiment de dents.
